# EUROPEAN PATENT APPLICATION

(11) **EP 1 775 277 A1**
(43) Date of publication of application: **18.04.2007**
(21) Application number: 05774297.5
(22) Date of filing: 25.05.2005
(51) Int. Cl.: C07C 5/27, B01J 29/04, B01J 29/80

(54) **METHOD AND CATALYST FOR THE TRANSALKYLATION/DEALKYLATION OF ORGANIC COMPOUNDS**

(30) Priority: 28.05.2004 ES 200401378
(71) Applicant: CONSEJO SUPERIOR DE INVESTIGACIONES CIENT FICAS, 28006 Madrid (ES); UNIVERSIDAD POLITECNICA DE VALENCIA, 46022 Valencia (ES)
(72) Inventor: CORMA CANÓS, A., Instituto de Tecnología Química, Avda. Los Naranjos, s/n, 46022 VALENCIA (ES); SERRA ALFARO, J.M., Instituto de Tecn. Quimica, Avda. Los Naranjos, s/n, 46022 Valencia (ES); FORN S SEGUÍ, V., Instituto de Tecnología Química, Avda. Los Naranjos, s/n, 46022 VALENCIA (ES); CASTAÑEDA SÁNCHEZ, R., Instituto de Tecn. Quimica, Avda. Los Naranjos, s/n, 46022 VALENCIA (ES)
(74) Representative: Ungria Lopez, Javier
(86) International application number: PCT/ES2005/070071
(87) International publication number: WO 2005/118515

(57) **Abstract**

The invention relates to a catalytic method for the transalkylation/dealkylation of organic compounds, consisting in bringing a supply comprising organic compounds into contact with a catalyst containing a first zeolitic component that is selected from among: a) one or more zeolites having crystalline structure ITQ-13; b) one or more zeolites having crystalline structure ITQ-13, which are modified either by means of selectivation or with the incorporation of one or more metals, or both; and c) a mixture of a) and b). The invention also relates to a catalyst comprising one or more modified zeolites having crystalline structure ITQ-13.

## Description

### FIELD OF APPLICATION

The invention relates to the use of solid catalysts for the transalkylation/dealkylation of organic compounds, preferably of alkylaromatic hydrocarbons, preferably of toluene and heavy fractions of reformed gasoline for producing xylenes.

### BACKGROUND

Aromatic products have a wide variety of applications in the chemical and petrochemical industry, such as, for example, the production of polyester monomers, advanced plastics, detergents, pharmaceutical products, agrochemical products, etc. Of all the aromatic products, benzene, toluene and xylenes (BTX) are the main starting products for many intermediate products.

The main sources of BTX formation are the catalytic naphtha pyrolysis and catalytic reforming processes. In these processes, the yields of each one of the BTX's are normally limited by the thermodynamic equilibrium. Specifically for the case of catalytic reforming, the BTX's are typically obtained in a 19/49/32 ratio. Therefore, there is a remarkable difference between the proportions in which each product is produced and the market demand. Thus, an excess of toluene is always produced by way of the reforming and pyrolysis processes, although there be a low demand for the same. On the other hand, there is a growing demand for benzene and xylenes. Hence, one process of economic interest is the transalkylation or disproportionation of toluene to produce benzene and xylenes, and/or the transalkylation of toluene with A9 (heavy reformed) nine-carbon-atom alkylaromatic compounds especially with trimethyl benzene (TMB) to yield benzene and xylenes, which are in high demand on the market. Therefore, this process makes it possible to transform toluene and nine-carbon-atom alkylaromatic products, an excess of both of which is produced in the catalytic reforming process, maximizing the yield of xylenes in the overall reforming process.

The para-xylene isomer is that in greatest demand due to its applications in the polymer industry.

### Toluene transalkylation

The main products of the transalkylation between two toluene molecules are benzene and xylenes, but different undesirable reactions such as the disproportionation of xylene, alkyl benzene dealkylation and cracking can occur. In the first industrial disproportionation processes, Friedel-Crafts (AlCl₃) catalysts were used, the xylenes being obtained in the thermodynamic equilibrium proportion, on which temperature has a slight influence. The equilibrium limits can be exceeded for the reaction when shape-selective zeolite catalysts having a selective molecular diffusion effect are used, especially when appropriately-processed ZSM-5 is used. In this regard, major degrees of selectivity for para-xylene have been achieved in commercial processes, with the corresponding positive impact on the process economics.

The basic principles for the improvement in para-selectivity include the reduction of the diffusivity and the inactivation of the centers of the zeolite crystal surface. The experimental techniques employed for achieving these effects include at least one of the following treatments:
- phosphorus treatment, with phosphorus contents of at least 0.5 percent by weight (US-4016219),
- treatment with oxides (US-4548914),
- selective carbonous deposit (coke) formation on the crystal surface - *precoking-* preferably when metals from Group IB to Group VIII had previously been added to the catalyst. (US-4097543)
- and depositing silica on the surface by means of different treatments, such as silylation with different organosiliceous or organonitrogenated silylating agents (US-4002697), tetra-alkyl-silicate vapor deposition (CVD) or treatment with silicones.

The primary product of the toluene disproportionation on the zeolitic catalysts is apparently p-xylene, whilst the secondary xylene isomerization reaction takes place at the zeolite pore mouth and on the external zeolite surface. Therefore, the inactivation of the external surface centers inhibits the secondary isomerization and makes it possible to maintain the selectivity of the products emerging from the internal zeolite pores. Likewise, the diffusivity in the zeolite varies to a great extent in terms of the molecular configuration, the para-xylene reaching a diffusion velocity in ZSM-5 of at least one thousand times faster than for all other isomers. However, the improvements in para-selectivity thanks to these characteristics implemented in the zeolite catalyst are achieved at the expense of reducing the overall catalytic activity.

For the purpose of improving different catalytic aspects, a standard practice is to incorporate metals into the zeolite. In the specific case of aromatic hydrocarbon-converting zeolite catalysts for producing xylenes (US-6,017,840), incorporating different metals, such as, for example, Pt, Ni or Re, into the zeolite structure makes it possible to improve the life of the catalyst, given that this makes it possible to reduce coke formation. However, the metal in the presence of hydrogen can saturate the aromatic ring, increasing the yield of undesirable by-products, as a result of which the metals must be incorporated carefully. The metal can be added to the catalyst by means of different techniques, such as aqueous-phase or solid-phase ion exchange, different impregnation methods, etc.

Processes of "selectivation" by formation of siliceous deposits are known in the state of the art and can be found, for example, in N.Y. Chen, Ind. Eng. Chem. Res. 40 (20) (2002) 4157-61, Lercher et al. Zeolites 17(3) (1994) 265-271, US-5726114.

A process consisting of treating a zeolite with phosphorated compounds has also been described, for example in Y. Xie et. al, ACS Symposium Series 738 (2000) 188-200 or US-4847435.

A process consisting of treating a zeolite with hydrocarbons such that carbonous deposits (coke) will be formed on the zeolite surface have likewise been described, for example in L-Y Fang et. al. J. Catal. 185, 33-42 (1999).

The typical conditions of the toluene disproportionation reactor are: pressure of 1-60 bar, temperature of 325-500°C, spatial velocity of 0.1 - 20 hours⁻¹ and hydrocarbon/toluene molar relation of 3 to 15.

Implementing zeolite ZSM-5 as a toluene disproportionation catalyst could be said to be a considerable improvement in the process. This improvement is due, to a great extent, to the topology and dimensions of the zeolite ZSM-5 pores. This zeolite has a system of two types of channels comprised of 10-membered rings, one running straight along the length of the crystal and measuring 5.1 x 5.5 , and the other running in zig-zag, crossing the first one and measuring 5.3 x 5.6 .

The catalyst to which this invention related contains a zeolite component (ITQ-13), the structure of which has been described by Corma et. Al in Angewandte Chemie, Int. Ed (2003), 42 (10), 1156-1159, and on which it has now been shown to have a high catalytic selectivity and activity for toluene disproportionation, making it possible to reduce, as compared to zeolite ZSM-5, the extending of undesirable cracking and xylene disproportionation reactions giving rise to trimethyl benzenes.

ITQ-13 synthetic zeolite has been described in different patents and publications (US-6,471,941; A. Corma et. Al., Angewandte Chemie, Int. Ed. (2003),42 (10), 1156-1159; US-2003171634; and J. Vidal-Moya et. Al., Chem. Mater. (2003), 15(21, 3961-3963). This zeolite structure has been recognized by the International Zeolite Association (IZA), having been assigned code ITH. This porous crystalline material consists of one single crystalline phase which has a three-way channel system comprised of three types of channels: (a) channels of ten-membered coordinated tetrahedral rings; b) generally parallel channels of ten-membered coordinated tetrahedral rings, which are perpendicular to and intersect with the type (a) channels; and (c) generally parallel nine-membered coordinated tetrahedral rings, which intersect with the type (a) and (b) channels. The cross-sectional dimensions of the type (a) channels are approximately 4.8 by 5.5, whilst the cross-sectional dimensions of the type (b) channels are approximately 5.0 by 5.7 . The cross-sectional dimensions of the type (c) channels are approximately 4.0 by 4.9 .

The ITQ-13 zeolite crystalline structure is defined by its single individual cell, which is the smallest structural unit containing all of the structural elements of this material. Table I shows the positions of each tetrahedral atom in the single cell, given in nanometers. Each tetrahedral atom is bound to an oxygen atom, which is bound to another adjacent tetrahedral atom. The position of each tetrahedron in the single individual cell may vary within a range of ±0.05 nanometers due to the mobility of these atoms resulting from vibrations or interactions with organic or inorganic species.

**Table I**

| | | | |
|---|---|---|---|
| T1 | 0.626 | 0.159 | 0.794 |
| T2 | 0.151 | 0.151 | 0.478 |
| T3 | 0.385 | 0.287 | 0.333 |
| T4 | 0.626 | 0.158 | 0.487 |
| T5 | 0.153 | 0.149 | 0.781 |
| T6 | 0.383 | 0.250 | 1.993 |
| T7 | 0.473 | 0.153 | 0.071 |
| T8 | 0.469 | 0.000 | 1.509 |
| T9 | 0.466 | 0.000 | 1.820 |
| T10 | 0.626 | 0.979 | 0.794 |
| T11 | 1.100 | 0.987 | 0.478 |
| T12 | 0.867 | 0.851 | 0.333 |
| T13 | 0.626 | 0.980 | 0.487 |
| T14 | 1.099 | 0.989 | 0.781 |
| T15 | 0.869 | 0.888 | 1.993 |
| T16 | 0.778 | 0.985 | 0.071 |
| T17 | 0.783 | 0.000 | 1.509 |
| T18 | 0.785 | 0.000 | 1.820 |
| T19 | 0.151 | 0.987 | 0.478 |
| T20 | 0.385 | 0.851 | 0.333 |
| T21 | 0.153 | 0.989 | 0.781 |
| T22 | 0.383 | 0.888 | 1.993 |
| T23 | 0.473 | 0.985 | 0.071 |
| T24 | 1.100 | 0.151 | 0.478 |
| T25 | 0.867 | 0.287 | 0.333 |
| T26 | 1.099 | 0.149 | 0.781 |
| T27 | 0.869 | 0.250 | 1.993 |
| T28 | 0.778 | 0.153 | 0.071 |
| T29 | 0.626 | 0.728 | 1.895 |
| T30 | 0.151 | 0.720 | 1.579 |
| T31 | 0.385 | 0.856 | 1.433 |
| T32 | 0.626 | 0.727 | 1.588 |
| T33 | 0.153 | 0.718 | 1.882 |
| T34 | 0.383 | 0.819 | 0.893 |
| T35 | 0.473 | 0.722 | 1.171 |
| T36 | 0.469 | 0.569 | 0.409 |
| T37 | 0.466 | 0.569 | 0.719 |
| T38 | 0.626 | 0.410 | 1.895 |
| T39 | 1.100 | 0.418 | 1.579 |
| T40 | 0.867 | 0.282 | 1.433 |
| T41 | 0.626 | 0.411 | 1.588 |
| T42 | 1.099 | 0.420 | 1.882 |
| T43 | 0.869 | 0.319 | 0.893 |
| T44 | 0.778 | 0.416 | 1.171 |
| T45 | 0.783 | 0.569 | 0.409 |
| T46 | 0.785 | 0.569 | 0.719 |
| T47 | 0.151 | 0.418 | 1.579 |
| T48 | 0.385 | 0.282 | 1.433 |
| T49 | 0.153 | 0.420 | 1.882 |
| T50 | 0.383 | 0.319 | 0.893 |
| T51 | 0.473 | 0.416 | 1.171 |
| T52 | 1.100 | 0.720 | 1.579 |
| T53 | 0.867 | 0.856 | 1.433 |
| T54 | 1.099 | 0.718 | 1.882 |
| T55 | 0.869 | 0.819 | 0.893 |
| T56 | 0.778 | 0.772 | 1.171 |

Zeolite ITQ-13 with unique topology and channel dimensions differing from zeolite ZSM-5 and from any other studied to date has been found to produce better results than those of zeolite ZSM-5 by increasing the selectivity for xylenes during the toluene disproportionation. Furthermore, it has been found that when this ITQ-13 zeolite is combined with large-pore zeolites, such as, for example beta or Y results in an active catalytic system selective for the dealkylation and transalkylation of alkylaromatic compounds of the heavy gasoline fraction reformed for forming benzene and xylenes.

### SUMMARY OF THE INVENTION

The invention relates, firstly, to a catalytic method for the transalklyation/dealkylation of organic compounds consisting of bringing a supply comprising organic compounds into contact with a catalyst containing a first zeolitic component that is selected from among:
a) one or more zeolites having crystalline structure ITQ-13,
b) one or more zeolites having crystalline structure ITQ-13 which are modified either by means of selectivation process, incorporation of one or more metals, or both, and
c) a combination of a) and b).

The above-mentioned organic compounds are preferably aromatic hydrocarbons. The supply in the above-mentioned catalytic method preferably consists of at least one of the following hydrocarbons: benzene, toluene, xylenes, ethyl benzene, trimethyl benzenes, ethyl toluenes, cumene, indane, tetramethyl benzene, diethyl benzene, butyl benzenes, ethyl xylenes, naphthalene, methyl naphthalene, anthracene, pentamethyl benzene, diethyl benzenes or diisopropyl benzene.

According to a preferred embodiment, the catalytic method of the invention is a process in which the transalkylation and/or dealkylation of toluene and nine-carbon-atom alkylaromatic hydrocarbons is produced to produce xylenes.

The catalytic method is preferably carried out under the following reaction conditions: (a) temperature within 250°C to 600°C range, preferably within 325°C to 500°C range; (b) pressure within 5 to 60 bar range, preferably within 15 to 45 bar range; (c) spatial velocity, given in kilograms of supply incorporated per kilogram of catalyst per hour, within 0.1 to 20 range, preferably within 0.5 to 12 range; and (d) a hydrogen / hydrocarbon molar relation of 2 to 25, preferably within the 3 to 15 range, a contact time of 0.05 to 10 hours.

A particularly preferred embodiment of the catalytic method according to the invention is the disproportionation of toluene to produce benzene and xylenes. The catalytic toluene disproportionation method is carried out for the purpose of maximizing the production of xylenes, especially of para-xylene. The production of undesirable by-products, such as ethyl benzene, trimethyl benzene and cracking by-products must be minimized, given that it remarkably reduces the process economics. Hence: (i) the end yield of xylenes and benzenes is reduced, (ii) molecules containing aromatic rings are destroyed, (iii) the final separation stages are hindered, the presence of ethyl benzene in the separation of the para-xylene being especially critical, and (iv) hydrogen is consumed when cracking reactions are carried out to produce short-chain paraffin hydrocarbons (C₁ - C₄).

The toluene disproportionation to produce benzene and xylenes is preferably carried out under the following reaction conditions: (a) temperature within 250°C to 600°C range; (b) pressure within 5 to 60 bar range; (c) spatial velocity within 0.1 to 20 kilograms of supply incorporated per kilogram of catalyst per hour range; (d) a hydrogen / hydrocarbon molar relation of 2 to 25; and (e) a contact time of 0.05 to 10 hours.

According to a preferred embodiment of the catalytic method of the invention, the catalyst is arranged on two or more catalytic beds, in which the composition of the zeolitic component of the catalyst is different in each one thereof.

According to a particularly preferred embodiment of the catalytic method of the invention, the catalyst is arranged on two or more catalytic beds:
- a first bed, in which the zeolitic component of the catalyst is selected from among a) and b), and
- a second bed, in which the zeolitic component of the catalyst is selected from among a) and b), and the above-mentioned second bed also comprises as the catalytic component one or more zeolites different from zeolite ITQ-13 and modified zeolite ITQ-13, and which are large-pore zeolites,.

According to a further alternative embodiment of the catalytic method of the invention, the composition of the catalyst varies along the length of catalytic bed, such that, on moving from the beginning to the end of the catalytic bed, one part of the zeolitic components of the catalyst constitutes a growing percentage by weight in relation to the catalyst and all of the other zeolitic components of the catalyst constitute a decreasing percentage by weight of the catalyst, in other words, in that same direction of movement, the percentage of one part of the zeolitic components progressively increases, whilst the percentage of all of the other catalytic components progressively decreases.

The composition of the catalyst varies preferably along the length of catalytic bed, such that, on moving from the beginning to the end of the reactor, a first zeolitic component selected from among a), b) and c), constitutes a growing percentage by weight of the catalyst, and a second zeolitic component comprised of one or more large-pore zeolites constitutes a decreasing percentage by weight of the catalyst.

In the catalytic method according to the invention, the zeolitic component a) is preferably zeolite of an ITQ-13 crystalline structure - zeolite ITQ-13 or ITH - comprising silicon and one or more T elements different from silicon, with a total Si/T atomic relation within 10 to 100 range.

The above-mentioned T element is preferably selected from among aluminum, gallium, boron, germanium, iron and combinations of same.

According to a preferred embodiment of the catalytic method, the above-mentioned zeolite of an ITQ-13 structure has aluminum as the T element, and the overall Si/Al atomic relation is within 10 to 100 range, the aluminum content of the zeolitic network being a minimum of 70% of the total aluminum content of the zeolite. This aluminum-containing zeolite of an ITQ-13 structure can have undergone an aluminum extraction process. Preferably for the disproportionation process and for the dealkylation-transalkylation process of aromatics from the reformed unit, the Si/Al molar relation in the ITQ-13 zeolite composition is within 10 to 100 range.

In the catalytic method of the invention, the above-mentioned zeolite of an ITQ-13 structure can be in acid form.

The above-mentioned ITQ-13 zeolite can also have undergone a process for the partial extraction of the T elements from the crystalline network.

According to a preferred embodiment of the catalytic method, the catalyst comprises a zeolitic component b) in which the ITQ-13 zeolite has been modified by means of a process selected from among selectivation, a process of incorporating one or more metals, or both processes.

At least one element selected from among the elements in Group IB, the elements in Group VIII and the elements of the groups included in between Group IB and Group VIII on the table can preferably be incorporated into the above-mentioned ITQ-13 zeolite, in an amount ranging from 0.01% to 10% by weight of each element.

Even more preferably, the above-mentioned element is selected from among Ag, Pt, Pd, Re, Ni, Mo, Ga, Bi, La, Cu and combinations of same.

The selectivation process is selected from among a zeolite crystal surface passivation process, a process of reducing the accessibility from the exterior of the crystals in the zeolite to the acid centers present in the pores inside the zeolite, and both processes.

The above-mentioned selectivation process can consists of depositing coke on the surface of the zeolite, obtaining a catalyst of a coke / zeolite mass ratio of at least 1/50.

The above-mentioned selectivation process can consists of depositing silica on the surface of the zeolite, obtaining a catalyst of a silica / zeolite mass ratio of at least 1/100.

The above-mentioned selectivation process can alternatively consists of treating the zeolite with phosphorated compounds, obtaining a catalyst with a phosphorus / zeolite mass ratio of at least 5/1000.

The above-mentioned selectivation process can alternatively consist of depositing coke or silica under conditions of a toluene disproportionation process in the presence of hydrogen.

In the catalytic method according to the invention, the catalyst, according to a preferred embodiment comprises: (i) a zeolitic component of an ITQ-13 structure in a quantity between 10% and 99.9% by weight, (ii) at least one element selected from among the elements in Group IB, the elements in Group VIII and the elements of the groups included in between Group IB and Group VIII on the periodic table, in an amount between 0.01% and 10% by weight of each element, and (iii) a matrix which comprises at least one material selected from alumina, silica, ceria, silica-alumina, zirconia, titania, and that constitutes the remainder of the composition of the catalyst. The aforesaid element (ii) is preferably selected from among one or more of Ag, Pt, Pd, Re, Ni, Mo, Ga, Bi, La and Cu.

According to a further preferred embodiment of the catalytic method, the catalyst comprises a first zeolitic component selected from among a), b) and c), and a second zeolitic component which is one or more large-pore zeolites, the aforementioned first zeolitic component and the aforementioned second zeolitic component being in a relation ranging from 0.5 to 20 by weight.

According to a further preferred embodiment of the catalytic method, the catalyst comprises: a first zeolitic component which is ITQ-13 and a second zeolitic component selected from among at least zeolite Beta (BEA), NU-87 (NES), SSZ-33 (CON), ITQ-7 (ISV), Mordenite (MOR), Y-zeolite (FAU), L-zeolite (LTL) and mazite (MAZ).

The present invention also relates to a zeolitic catalyst characterized in that it comprises zeolite of an ITQ-13 crystalline structure, modified by means of a process selected from among a selectivation process, a process of incorporating one or more metals, and a combination of the two.

According to a preferred embodiment of the catalyst, the aforementioned ITQ-13 zeolite is modified by means of the incorporation of at least one element selected from among the elements in Group IB, the elements in Group VIII and the elements of the groups included in between Group IB and Group VIII on the periodic table, in an amount ranging from 0.01% to 10% by weight of each element.

Yet more preferably, the aforementioned element is selected from among Ag, Pt, Pd, Re, Ni, Mo, Ga, Bi, La, Cu and combinations of same. This modification by means of the addition of metals affords the possibility of lengthening the lifetime of the catalyst, given that the speed of the coke deposition on the inside of the microporous system of the zeolitic component of the catalyst is slowed down. However, the type of metal, the amount to be added and the incorporation method must be carefully selected, given that the metal can favor the forming of undesirable reaction by-products, or the metal may rather progressively lose its activity on being exposed to high temperatures during the different catalyst preparation, regeneration treatments, etc. For example, platinum tends to clump at the usual temperatures at which the calcination operations are carried out, such that the area of the platinum particles is reduced and its catalytic properties are seriously altered.

The aforementioned elements of Groups IB to Group VIII are incorporated by methods well-known in the art, such as impregnation or by ion exchange.

In the catalyst of the invention, the zeolitic component of an ITQ-13 crystalline structure can alternately be modified by means of a process consisting of passivating the surface of the zeolite crystals; a process which consists of reducing the accessibility from the exterior of the zeolite crystals to the acid centers present in the internal pores of the zeolite; and/or a process for modifying the diffusivity of the different alkylaromatic isomers. The aforementioned processes are termed zeolite "selectivation" processes. These selectivation processes can be carried out under the same conditions under which the disproportionation reaction of alkylaromatic hydrocarbons such as toluene is carried out.

The aforementioned "selectivation" processes make it possible to increase the catalytic selectivity of the zeolite for the para-isomers of dialkylaromatic molecules in the disproportionation reaction of an alkylaromatic compound such as toluene.

One of the processes worthy of mention making it possible to increase the selectivity for the para-isomers is a process comprising depositing coke onto the surface of an ITQ-13 zeolite, obtaining a catalyst with a coke / zeolite mass ratio of at least 1/50.

One of the processes worthy of mention making it possible to increase the selectivity for *para*-isomers is a process comprising depositing silica on the surface of an ITQ-13 zeolite, obtaining a catalyst with a silica / zeolite mass ratio of at least 1/100. In this process, the treatment can be carried out in the gas phase or liquid phase. Organosiliceous compounds or silicones can be used, which are preferably deposited on the surface of the zeolite crystal and then can be broken down to form siliceous deposits on the material undergoing heat treatments such as airflow calcination. The final silica content deposited on the zeolite has to be at least 1% of the total mass of the treated zeolite. These "selectivation" processes by formation of siliceous deposits have been described in the documents to which reference is made in the section of this document devoted to Background information.

One of the processes also worthy of mention making it possible to increase the selectivity for para-isomers is a process consisting of treating the zeolite with phosphorated compounds such that a phosphorous / zeolite mass ratio of at least 5/1000 is obtained. By means of this process, the zeolite surface is irreversibly coated with phosphorated compounds.

One of the processes also worthy of mention making it possible to increase the selectivity for para-isomers is a process consisting on depositing coke or silica of a toluene disproportionation process condition in the presence of hydrogen.

One of the processes also worthy of mention making it possible to increase the selectivity for para-isomers is a process comprising treating the ITQ-13 zeolite with hydrocarbons such that carbonous (coke) deposits are formed on the zeolite surface.

According to one preferred embodiment, the catalyst consists of: (i) a zeolitic component of an ITQ-13 structure in an amount ranging from 10% to 99.9% by weight, (ii) at least one element selected from among the elements in Group IB, the elements in Group VIII and the elements included in between Group IB and Group VIII on the periodic table, in an amount ranging from 0.01% to 10% by weight of each element and (iii) a matrix consisting of at least one material selected from among alumina, silica, ceria, silica-alumina, zirconia, titania and that constitutes the remainder of the composition of the catalyst. The zeolitic component of this catalyst preferably has a Si/Al ratio between 10 to 100 The aforementioned element selected from among the elements in the groups included in between Group IB and Group VIII on the periodic table is preferably selected from among Ag, Pt, Pd, Re, Ni, Mo, Ga, Bi, La and Cu.

According to one preferred embodiment, the catalyst consists of:
- a first zeolitic component selected from among:
   - an ITQ-13 zeolite,
   - zeolite of a modified ITQ-13 crystalline structure and
   - a combination of both of the above;
- and a second zeolitic component which is at least one large-pore zeolite, said first and second zeolitic component being in a ratio ranging from 0.5 to 20 by weight.

The above-mentioned large-pore zeolite is a zeolite (ZPG) having pores consisting of rings comprised of 12 or more members, such as, for example, beta zeolite, SSZ-33 or combinations of the same. The above-mentioned second zeolitic component can be selected from among at least Beta zeolite (BEA), NU-87 (NES), SSZ-33 (CON), ITQ-7 (ISV), Mordenite (MOR), Y-zeolite (FAU), L-zeolite (LTL) and mazite (MAZ). The ITQ-13 to ZPG ratio is preferably within 0.5 and 20 by weight, being especially suitable for producing benzene and xylenes from toluene and heavy gasoline fractions. According to this alternative embodiment, this catalyst is used in a fixed-bed reactor in which the different zeolites are used combined on one single bed or in different areas of the catalytic bed.

The catalyst of the present invention is preferably in the form of pellets or balls, which can be conformed by extrusion or compression.

The catalyst of the invention can be put through a final calcination at a temperature within 250°C to 600°C range.

As previously described hereinabove, the ITQ-13 zeolite can form part of the catalyst of this invention. It is preferably comprised of silicon and at least one T element, with an overall Si/T atomic relation within 10 to 100 range. T is preferably selected from among aluminum, gallium, boron, germanium, iron and combinations thereof.

More preferably, the aforementioned ITQ-13 zeolite comprises silicon and at least one T element which is aluminum and has an overall Si/Al atomic relation within 10 to 100 range, the contents of the zeolite network being a minimum of 70% of the total aluminum content in the zeolite.

Zeolite ITQ-1 3 can also be in the catalyst in acid form.

In the catalyst of the invention, the aforementioned ITQ-13 crystalline structure zeolite comprises T elements can have undergone a process of partially extracting the T elements from the crystalline network.

The aforementioned T element extraction process can consist of at least one treatment selected between a calcination treatment in the presence of water vapor and a treatment with organic acids, inorganic acids or chelating compounds.

In the catalyst of the present invention, the zeolite of an ITQ-13 structure that comprises aluminum as on of the T elements can also have undergone an aluminum extraction process.

### BRIEF DESCRIPTION OF THE FIGURES

Figure 1 shows the powder X-ray diffraction diagram for the powder zeolite ITQ-13 of the catalyst prepared according to Example 1 - ITQ-13 zeolite -.
Figure 2 shows the toluene conversion achieved in the toluene disproportionation test with catalysts A, B, C and D - examples 1, 6, 7 and 8 - the percentage toluene conversion (%X) being shown on the y-axis and the temperature (°C) on the x-axis.
Figure 3 shows the molar yield of trimethyl benzene (y-axis) as compared to the conversion of toluene (x-axis) for catalysts A, B, D and E - examples 1, 6, 8 and 9.
Figure 4 represents the molar yield of cracking products C₁- C₄ (y-axis) as compared to the toluene conversion (x-axis) for catalysts A, B, D and E.

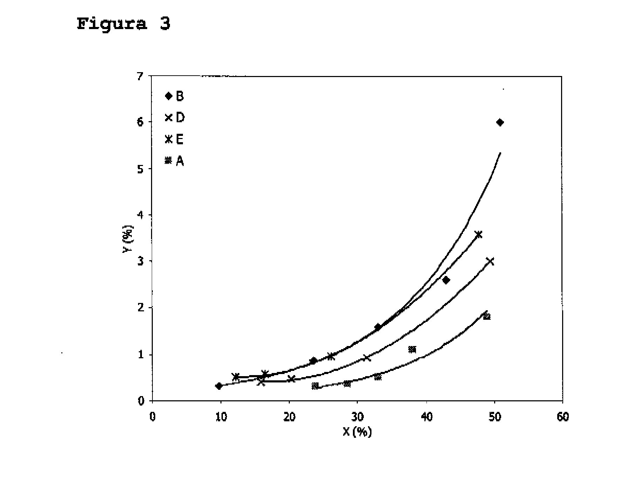

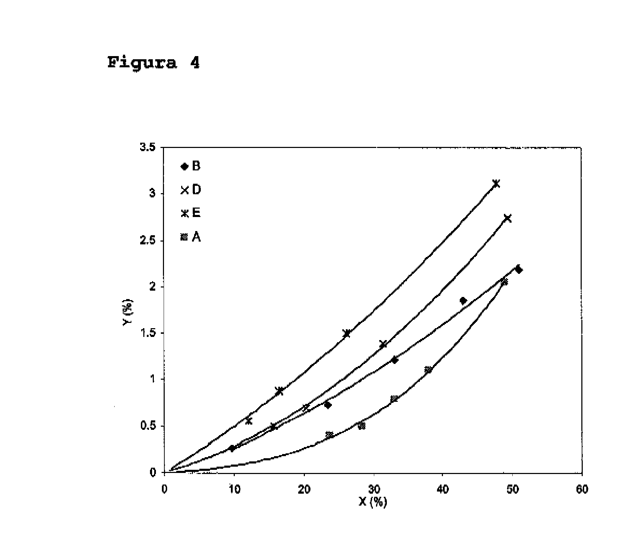

The following examples are provided for the purpose of illustrating the aspects of the invention but in no way limit the scope thereof.
**Example 1.** Preparation of an ITQ-13 zeolite
The synthesis of an ITQ-13 zeolite was carried out as follows: 0.242 grams of aluminum isopropoxide are added onto 11.2 grams of tetraethylorthosilicate (TEOS). Next, 43 grams of a hexametonium dihydroxide (SDA) solution containing 0.77 equivalents of hydroxide in 1000 grams are added. To this mixture, 0.56 grams of germanium oxide are added and allowed to evaporate under stirring to complete evaporation of the ethanol from the TEOS hydrolysis plus the amount of water necessary to achieve the following final composition: 0.91 SiO₂ : 0.09 GeO₂ : 0.01 Al₂O₃ : = 0.28 SDA:0.56 HF:7H₂O. Lastly, 1.38 grams of a fluorhydric acid solution (48% HF by weight) are added. The resulting mixture is placed inside polytetrafluorethylene-lined autoclaves and heated to 135°C for 21 days under agitation. This material was calcinated in a mufla oven to 540° for 2 hours, with a heating ramp of 1°C/min and an intermediate calcination stage at 300°C for 2 hours. The final material obtained contained less than 0.5% organic matter and was in acid form, being referred to from this point onward as catalyst A.

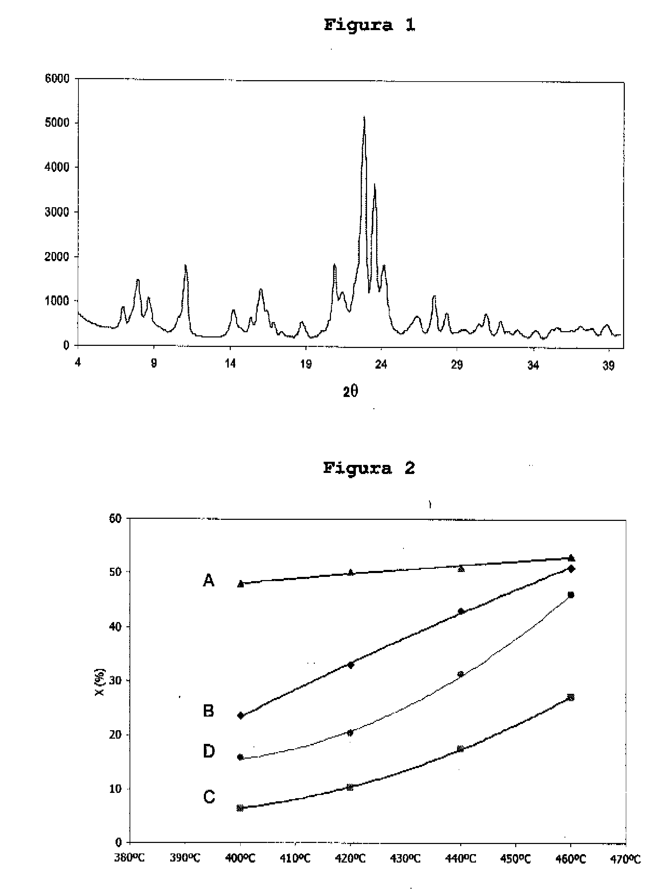

Figure 1 shows the powder X-ray diffraction diagram for catalyst A, taken using Kₐ copper radiation in Phillips PW1830 diffractometer. The spectrum in Figure 1 corresponds to the X-ray diffraction pattern characteristic of the ITQ-13 zeolite structure.
**Example 2.** Catalytic activity for toluene disproportionation of the zeolite described in Example 1.
The ITQ-13 zeolite (Catalyst A) in Example 1 was conformed in particles of diameters within the 0.2 - 0.5 mm range, by means of forming tablets by compressing, grinding and sifting. 1 gram of this material is mixed with silicon carbon particles ranging from 0.6 to 0.9 mm in size up to the point at which the volume of the mixture of the two was 2.5 ml. The homogeneous mixture of these two materials was placed inside a steel tube reactor of a one-inch outer diameter. The catalytic toluene disproportionation experiment was conducted under the following reaction conditions: pressure 30 bar, spatial velocity , 1 hour⁻¹ hydrogen / toluene molar relation of 8.5 and 400°C temperature. Under these conditions, following a six-hour reaction time, a 47.9% conversion was achieved, with a molar yield of benzene and xylenes respectively of 23.5% and 21.1%.
**Example 3.** Catalyst A was studied using the same experimental method and reaction conditions as in Example 2, the only modification being the reaction temperature, which was 420°C in this case. Under these conditions, a 50.3% conversion was achieved, with a molar yield of benzene and xylenes respectively of 24.2% and 21.8%.
**Example 4.** Catalyst A was studied using the same experimental method and reaction conditions as in Example 2, modifying the reaction temperature, which was 440°C in this case. Under these conditions, a 51.1% conversion was achieved, with a molar yield of benzene and xylenes respectively of 24.5% and 21.7%.
**Example 5**. Catalyst A was studied using the same experimental method and reaction conditions as in Example 2, modifying the reaction temperature, which was 460°C in this case. Under these conditions, a 53.1% conversion was achieved, with a molar yield of benzene and xylenes respectively of 25.3% and 22.2%.
**Example 6.** A catalyst (B) comprised of ZSM-5 zeolite in its acid form with a Si/Al ratio of 17.5, supplied by PQ Corporation with code CBV-3020, was used exactly as described in Example 2, a toluene conversion of 23.5% being obtained after a six-hour reaction time, with a molar yield of benzene and xylenes respectively of 11.3% and 10.6%
**Example 7.** A catalyst (C) comprised of ZSM-5 zeolite in its acid form with a Si/Al ratio of 32, supplied by PQ Corporation with code CBV-8020, was used exactly as described in Example 2, a 6.5% toluene conversion being obtained after a six-hour reaction time with a molar yield of benzene and xylenes respectively of 3.1% and 3%.
**Example 8.** A catalyst (D) comprised of Mordenite zeolite in its acid form with Si/Al ratio of 10, supplied by PQ Corporation with code CBV-20A, a 39.1% toluene conversion being obtained after a six-hour reaction time, with a molar yield of benzene and xylenes respectively of 19.8% and 17.1%.
Catalysts B, C and D were studied using the same experimental method and reaction conditions as in Example 2, the only modification being the reaction temperature, such that three different temperatures, 420°C, 440°C and 460°C, were studied. The toluene conversion obtained in the toluene disproportionation test with catalysts A, B, C and D is provided in Figure 2, showing the percentage toluene conversion (%X) on the y-axis and the temperature (°C) on the x-axis. For all of the temperatures tested in the catalytic reactor, the toluene conversion obtained with disproportionation catalyst A in accordance with the present invention is clearly greater than the conversions obtained with catalysts B, C and D, which are not in agreement with the invention. Therefore, for catalyst A, according to the present invention, a toluene conversion nearing that of thermodynamic equilibrium at a temperature of 420°C is achieved, which for this range of temperatures is of approximately 53%, whilst with all of the other catalysts, not furnished by the present invention, the conversions achieved at 420°C are at least 20% of the equilibrium conversion.
**Example 9.** An E catalyst was prepared using Beta zeolite in its acid form with a Si/Al ratio of 12.5, supplied by PQ Corporation with code CP-811. Catalysts A, B, D and E were put through a toluene disproportionation test in the reaction system previously described hereinabove under the following conditions: constant temperature 425°C, pressure 30 bar, hydrogen /toluene molar relation of 8.5, and the spatial velocity was variable from 1 to 12 hr⁻¹ such that it was possible to achieve different degrees of conversion for each catalyst. Figure 3 shows the molar yield of trimethyl benzene (y-axis) as compared for the toluene conversion (x-axis) for catalysts A, B, D and E, Catalyst A is that which makes it possible to obtain a smaller amount of the undesirable reaction product, by increasing the selectivity of the process toward the production of xylenes. Likewise, Figure 4 shows the molar yield of cracking products C₁-C₄ (y-axis) as compared to the toluene conversion (x-axis) for catalysts A, B, D and E. In this case, with catalyst A, the lowest yields of undesirable cracking products are obtained, by once again increasing the selectivity toward xylenes. With all of the catalysts tested, the same proportion was found to exist among the xylene ortho-, meta- and para-isomers, corresponding to that dictated by thermodynamic equilibrium. Therefore, catalyst A according to the invention shows a high catalytic activity and selectivity toward the production of benzene and xylenes.
**Example 10.** A catalyst F was prepared by wet impregnation of catalyst E with ammonium perrenate and then drying at 100°C for 8 hours, such that the end rhenium content was 0.3%. This catalyst was conformed in particles of diameters of 0.2 - 0.5 mm by means of forming tablets by compressing, grinding and sifting. 1 gram of this material was mixed with silicon carbon particles ranging in size from 0.6 to 0.9 mm until the volume of the mixture of the two reached 2.5 ml. The homogeneous mixture of these two materials was placed inside a steel tube reactor having a one-inch outer diameter. Next, the catalyst was reduced under hydrogen-flow at 450°C for two hours. The catalytic transalkylation experiment was conducted using a load constituted by 50% toluene, 16% ethyl toluene, 28% trimethyl benzene and 6% aromatic hydrocarbons of ten or more carbon atoms as the supply. The conditions of the catalytic experiment were as follows: temperature 400°C, total pressure 25 bar, spatial velocity WHSV 4 hr⁻¹ and hydrogen to hydrocarbon ratio of 8.5 mol/mol. The results of the treatment of the load described hereinabove obtained with the F catalyst are shown in Table 2.

| | Catalyst F | Catalyst H |
|---|---|---|
| Yield % xylenes | 25.9 | 26.4 |
| Yield % benzenes | 5.5 | 5.5 |
| Conversion % Trimethyl benzenes | 52.8 | 53.5 |
| Conversion % Ethyl toluenes | 67.0 | 68.3 |
| Conversion % Propyl benzenes | 93.4 | 95.6 |
| Conversion % Ethyl xylenes | 10.5 | 17.3 |
| Yield % A₁₀ aromatics | 3.2 | 2.9 |
| Yield % A₁₁⁺ aromatics | 0.54 | 1.42 |
| Yield % Ethyl benzene | 1.9 | 1.7 |
| Yield % Methane | 0.12 | 0.04 |
| Yield % Ethane | 1.80 | 1.85 |
| Yield % Propane | 3.45 | 1.46 |
| Yield % Butanes and Pentanes | 4.21 | 0.98 |

**Example 11.** A catalyst G was prepared by wet impregnation of catalyst A with ammonium perrenate and then dried at 100°C for 8 hours, such that the final rhenium contents was 0.3%. Next, catalysts F and G were mixed in powder form in equal proportions, obtaining catalyst H, in accordance with the present invention. Catalyst H was put through the experiment described in Example 10, such that the same spatial velocity with respect to Catalyst G content in the catalytic bed was maintained. The results obtained with catalyst H are shown in Table 2.
With catalyst H, in accordance with the present invention, an interesting improvement is achieved in the final yield of xylenes and in the conversion of trimethyl benzenes, ethyl toluenes and propyl benzenes. Likewise, the amount of by-products of the catalytic process, such as ethyl benzene and especially cracking gases which decrease the selectivity of the process and produce an additional consumption of hydrogen, is remarkably reduced.
**Example 12.** A catalyst I was prepared by wet impregnation of catalyst A with ammonium perrenate and then drying at 100°C for 8 hours, such that the final rhenium content was 0.3%. This catalyst was conformed in particles of diameters between 0.2 and 0.5 mm, by means of tablet forming by compressing, grinding and sifting. This catalyst was put through a disproportionation experiment exactly the same as that described in Example 2, a 56% toluene conversion being obtained after six-hour reaction time, with a molar yield of benzene, xylenes and cracking products respectively of 24.3%, 23.6% and 4.0%.

## Claims

1. A catalytic method for transalkylaton/dealkylation of organic compounds consisting of bringing a supply comprising organic compounds into contact with a catalyst comprising a first zeolitic component selected from among:
a) one or more zeolites of ITQ-13 crystalline structure,
b) one or more zeolites of ITQ-13 crystalline structure, modified by means of a process selected from among selectivation, incorporation of one or more metals or both thereof, and
c) a combination of a) and b).

2. A catalytic method according to claim 1, in which the supply of organic compounds is a aromatic hydrocarbon supply.

3. A catalytic method according to claim 2, in which the aforementioned aromatic hydrocarbons are selected from among at least one of: benzene, toluene, xylenes, ethyl benzene, trimethyl benzenes, ethyl toluenes, cumene, indane, tetramethyl benzene, diethyl benzene, butyl benzenes, ethyl xylenes, naphthalene, methyl naphthalene, anthracene, pentamethyl benzene, diethyl benzenes and diisopropyl benzene.

4. A catalytic method according to claim 1, **characterized in that** a transalklyation reaction of nine-carbon-atom alkylaromatic hydrocarbons is carried out to produce xylenes.

5. A catalytic method according to one of claims 1 to 4, **characterized in that** it is carried out under the following reaction conditions: (a) temperature range of 250°C to 600°C, (b) pressure range of 5 to 60 bar, (c) spatial velocity within the range of 0.1 to 20 kilograms of supply incorporated per kilogram of catalyst per hour, (d) a hydrogen/hydrocarbon molar relation of 2 to 25 and (e) a contact time between 0.05 and 10 hours.

6. A catalytic method according to claim 1, **characterized in that** the disproportionation of toluene is produced to produce benzene and xylenes.

7. A catalytic method according to claim 1, **characterized in that** the disproportionation of toluene is produced to produce benzene and xylenes under the following reaction conditions: (a) temperature range of 250°C to 600°C, (b) pressure range of 5 to 60 bar, (c) spatial velocity within the range of 0-1 to 20 kilograms of supply incorporated per kilogram of catalyst per hour, (d) a hydrogen/hydrocarbon molar relation of 2 to 25 and (e) a contact time between 0.05 and 10 hours.

8. A catalytic method according to one of claims 1 to 7, **characterized in that** the catalyst is arranged on two or more catalytic beds, on which the composition of the zeolitic component of the catalyst is different in each one of said catalytic beds.

9. A catalytic method according to claim 8, **characterized in that** the catalyst is arranged on two catalytic beds,
- a first bed on which the zeolitic component of the catalyst is selected between a) and b), and
- a second bed on which the zeolitic component of the catalyst is selected between a) and b), and said second bed is also comprised of one or more large-pore zeolites as a catalytic component.

10. A catalytic method according to one of claims 1 to 9, **characterized in that** the composition of the catalyst varies along the length of the catalytic bed, such that, on moving from the beginning to the end of the catalytic bed, one part of the zeolitic components of the catalyst constitute a growing percentage by weight of the catalyst, and the remainder of the zeolitic components of the catalyst constitute a decreasing percentage by weight of the catalyst.

11. A catalytic method according to claim 1, **characterized in that** the composition of the catalyst varies along the length of the catalytic bed, such that, on moving from the beginning to the end of the reactor, a first zeolitic component selected from among a), b) and c) constitutes a growing percentage by weight of the catalyst, and a second zeolitic component comprised of one or more large-pore zeolites constitutes a decreasing percentage by weight of the catalyst.

12. A catalytic method according to claim 1, **characterized in that** the zeolitic component a) is zeolite of an ITQ-13 crystalline structure comprising silicon and one or more T elements other than silicon, with a total Si/T atomic relations within 10 to 100 range.

13. A catalytic method according to claim 12, in which at least one T element is selected from among aluminum, gallium, boron, germanium, iron and combinations thereof.

14. A catalytic method according to claim 12, in which said ITQ-13 structure zeolite has aluminum as the T element, and the overall Si/Al atomic relation is within 10 to 100 range, the aluminum content of the zeolitic network being at least 70% of the total aluminum content of the zeolite.

15. A catalytic method according to claim 12, in which the aforementioned ITQ-13 structure zeolite comprises aluminum and has undergone an aluminum-extraction process.

16. A catalytic method according to claim 1, in which said ITQ-13-structure zeolite is in acid form.

17. A catalytic method according to claim 12, in which said ITQ-13-structure zeolite has undergone a process to partially extract the T elements from the crystalline network.

18. A catalytic method according to claim 11, **characterized in that** the ITQ-13 zeolite is modified by means of the incorporation of at least one element selected from among the elements in Group IB, the elements in Group VIII and the elements included in between Group IB and Group VIII on the periodic table, in an amount ranging from 0.01% to 10% by weight of each element.

19. A catalytic method according to claim 18, **characterized in that** said element is selected from among Ag, Pt, Pd, Re, Ni, Mo, Ga, Bi, La, Cu and combinations of same.

20. A catalytic method according to claim 1, **characterized in that** the selectivation process is selected from among a zeolite crystal surface passivation process, a process for the reduction of the accessibility from the exterior of the zeolite crystals to the acid centers in the interior zeolite pores, and both of these two processes.

21. A catalytic method according to claim 20, **characterized in that** the selectivation process comprises depositing coke onto the zeolite surface, obtaining a catalyst with a coke / zeolite mass ratio of at least 1/50.

22. A catalytic method according to claim 20, **characterized in that** the selectivation process comprises depositing silica on the zeolite surface, obtaining a catalyst with a silica / zeolite mass ratio of at least 1/100.

23. A catalytic method according to claim 20, **characterized in that** the selectivation process consists of treating the zeolite with phosphorated compounds, obtaining a catalyst with a phosphorus / zeolite mass ratio of at least 5/1000.

24. A catalytic method according to claim 20, **characterized in that** the selectivation process consists of depositing coke or silica under conditions of a toluene disproportionation process in the presence of hydrogen.

25. A catalytic method according to claim 1, **characterized in that** the catalyst comprises: (i) an ITQ-13-structure zeolitic component in an amount ranging from 10% to 99.9% by weight, (ii) at least one element selected from among the elements in Group IB, the elements in Group VIII and the elements in the groups included in between Group IB and Group VIII on the periodic table, in an amount ranging from 0.01% to 10% by weight of each element, and (iii) a matrix comprising at least one material selected from among alumina, silica, ceria, silica-alumina, zirconia, titania, and that constitutes remainder of the contents of the catalyst.

26. A catalytic method according to claim 25, **characterized in that**, at least one element selected from among Ag, Pt, Pd, Re, Ni, Mo, Ga, Bi, La and Cu.

27. A catalytic method according to claim 1, **characterized in that** the catalyst comprises of a first zeolitic component selected from among a), b) and c), and a second zeolitic component which is one or more large-pore zeolites, said first zeolitic component and said second zeolitic component having a relation of 0.5 - 20 by weight.

28. A catalytic method according to claim 1, **characterized in that** the catalyst comprises: a first zeolitic component which is ITQ-13 and a second zeolitic component selected from among at least Beta zeolite (BEA), NU-87 (NES), SSZ-33 (CON), ITQ-7 (ISV), Mordenite (MOR), Y-zeolite (FAU), L-zeolite (LTL) and mazite (MAZ).

29. A zeolitic catalyst **characterized** because it comprises ITQ-13 crystalline structure zeolite modified by means of a process selected from among a selectivation process, a process of incorporating one or more metals, and a combination of the two.

30. A catalyst according to claim 29, which is ITQ-13 zeolite modified by means of the incorporation of at least one element selected from among the elements in Group IB, the elements in Group VIII and the elements in the groups included in between Group IB and Group VIII on the periodic table, in an amount ranging from 0.01 % to 10% by weight of each element.

31. A catalyst according to claim 30, in which said element is selected from among Ag, Pt, Pd, Re, Ni, Mo, Ga, Bi, La, Cu and combinations of the same.

32. A catalyst according to claim 29, which is ITQ-13 zeolite modified by means of a selectivation process selected from among a zeolite crystal surface passivation process, a process of reducing the accessibility from the exterior of the zeolite crystal to the acid centers present in the interior zeolite pores, and both of these two processes.

33. A catalyst according to claim 32, which is ITQ-13 zeolite modified by means of a selectivation process consisting of depositing coke onto the zeolite surface, obtaining a catalyst with a coke / zeolite mass ratio of at least 1/50.

34. A catalyst according to claim 32, which is ITQ-13 zeolite modified by means of a selectivation process consisting of depositing silica on the ITQ-13 zeolite surface, obtaining a catalyst with a silica / zeolite mass ratio of at least 1/100.

35. A catalyst according to claim 32, which is ITQ-13 zeolite modified by means of a selectivation process consisting of treating the ITQ-13 zeolite with phosphorated compounds, obtaining a catalyst with a phosphorous / zeolite mass ratio of at least 5/1000.

36. A catalyst according to claim 32, which is ITQ-13 zeolite modified by means of a selectivation processing consisting of depositing coke or silica under conditions of a toluene disproportionation process in the presence of hydrogen.

37. A catalyst according to claim 29, **characterized in that** it comprises: (i) a ITQ-13-structure zeolitic component in an amount ranging from 10% to 99.9% by weight, (ii) at least one element selected from among the elements in Group IB, the elements in Group VIII and the elements in the groups included in between Group IB and Group VIII on the periodic table in an amount ranging from 0,01% and 10% by weight of each element and (iii) a matrix comprising at least one material selected from among alumina, silica, ceria, silica-alumina, zirconia, titania and that constitutes the remainder of the composition of the catalyst.

38. A catalyst according to claim 37, **characterized in that** (ii) is at least one element selected from among Ag, Pt, Pd, Re, Ni, Mo, Ga, Bi, La and Cu.

39. A catalyst according to any one of claims 29 to 38, **characterized in that** it comprises a first zeolitic component selected from among:
- an ITQ-13 zeolite,
- modified ITQ-13 crystalline structure zeolite and
- a combination of same;
- and a second zeolitic component which is at least one large-pore zeolite, said first and second zeolitic component being in a relation ranging form 0.5 to 20 by weight.

40. A catalyst according to claim 39, in which said first zeolitic component is ITQ-13 and said second zeolitic component is selected from among at least Beta zeolite (BEA), NU-87 (NES), SSZ-33 (CON), ITQ-7 (ISV), Mordenite (MOR), Y-zeolite (FAU), L-zeolite (LTL) and mazite (MAZ).

41. A catalyst according to any one of claims 29 to 40, **characterized in that** it has undergone a final calcination at a temperature within the 250°C - 600°C range.
